(19) Europäisches Patentamt European Patent Office Office européen des brevets

(11) EP 2 172 116 B1

(12) EUROPEAN PATENT SPECIFICATION

(45) Date of publication and mention of the grant of the patent:
07.06.2017 Bulletin 2017/23

(51) Int Cl.:
A23L 7/00 (2016.01)          A23L 7/10 (2016.01)
A23L 33/105 (2016.01)        A61K 36/899 (2006.01)

(21) Application number: 08740682.3

(22) Date of filing: 18.04.2008

(86) International application number:
PCT/JP2008/057636

(87) International publication number:
WO 2009/011158 (22.01.2009 Gazette 2009/04)

(54) **RICE BRAN-LIKE COMPOSITION AND FOOD**

REISKLEIEARTIGE ZUSAMMENSETZUNG UND NAHRUNGSMITTEL

COMPOSITION ET ALIMENT ANALOGUE AU SON DE RIZ

(84) Designated Contracting States:
AT BE BG CH CY CZ DE DK EE ES FI FR GB GR HR HU IE IS IT LI LT LU LV MC MT NL NO PL PT RO SE SI SK TR

(30) Priority: 18.07.2007 JP 2007187656

(43) Date of publication of application:
07.04.2010 Bulletin 2010/14

(73) Proprietor: SUNSTAR INC.
Takatsuki-shi,
Osaka 569-1195 (JP)

(72) Inventors:
• KAWASE, Ren
Takatsuki-shi
Osaka 569-1195 (JP)
• AZAMI, Shouji
Tokyo 124-8530 (JP)
• MAEDA, Yoshiaki
Tokyo 124-8530 (JP)

(74) Representative: TBK
Bavariaring 4-6
80336 München (DE)

(56) References cited:
WO-A1-2007/029631    WO-A2-2007/060947
JP-A- 2005 143 364    JP-A- 2007 068 523
JP-A- 2007 185 156

• A. A.,HAMID, Y. S., LUAN: FOOD CHEMISTRY, no. 68 5 May 1999 (1999-05-05), pages 15-19, XP002664741, Retrieved from the Internet: URL:http://www.mendeley.com/research/funct ional-properties-of-dietary-fibre-prepared -from-defatted-rice-bran/#page-1 [retrieved on 2011-11-30]
• YOSHIAKI MAEDA ET AL.: 'Handasshi Komenuka Kako Shokuhin ga Koshiboshoku Rat no Tainai Shiboryo Narabini Kecchu Shishitsu ni Oyobosu Eikyo' 61ST THE JAPANESE SOCIETY OF NUTRITION AND FOOD SCIENCE TAIKAI KOEN YOSHISHU 20 April 2007, page 161
• KAHLON, T. S. ET AL.: 'Cholesterol lowering effects of rice bran and rice bran oil fractions in hypercholesterolemic hamsters' CEREAL CHEM. vol. 69, no. 5, 1992, pages 485 - 489, XP008128977

**Description**

[Technical Field]

The present invention relates to a use of a rice bran-like composition and a food.

[Background Art]

Rice bran comprises the pericarp, seed coat, perisperm, aleurone layer, etc. of rice, which are obtained as by-products when brown rice is polished to produce white rice. Rice bran is known to be rich in oil, proteins, minerals, and Vitamin B family (particularly $B_1$). Further, $\gamma$-oryzanol contained in rice bran is known to have an antioxidant effect and a cholesterol lowering effect. Accordingly, there have been attempts to use rice bran products as supplements and food additives with a purpose of enhancing nutrient content or providing the above-mentioned effects (for example, see Patent Document 1).

Rice obtained by polishing may be referred to as polished white rice, 70% polished rice, 50% polished rice (half- polished rice), or 30% polished rice, depending on the degree of bran removal. Additionally, rice bran contains a lipase, and turns rancid very quickly as a result of the action of the lipase. Accordingly, in many cases, rice bran is dried by heating in order to deactivate the lipase to improve the stability of rice bran.

Because rice bran generally contains 15 to 20% of oil, rice bran is used as a raw material for rice bran oil (rice oil). Previously, crude rice bran oil was obtained by pressing rice bran. However, the yield was poor because oil would remain in the pressed residue. Accordingly, today, for the purpose of producing rice bran oil, crude oil is commonly obtained by a method in which rice bran is defatted by solvent (e.g., hexane and acetone) extraction.

Previously, extraction residues (defatted rice bran) produced by solvent extraction of rice bran were used as forage. However, today, the extraction residues are not used much as forage because of safety concerns associated with residual solvent. Yet, because useful components are contained in the extraction residues, the extraction residues are used as raw materials for isolating useful components therefrom. Further, a recent report includes the use of defatted rice bran by processing defatted rice bran into powders for use as food additives for enhancing nutrient content (for example, see Patent Document 2).

When using crude oil obtained by pressing or solvent extraction as edible rice bran oil, the rice bran oil is produced by refining crude oil through processes such as degumming, dewaxing, deoxidizing, decoloring, deodorizing, etc.

[Patent Document 1] Japanese Unexamined Patent Publication No. 2007-068523
[Patent Document 2] Japanese Unexamined Patent Publication No. 2007-104962

JP 2005 143364 A describes a cooking method for cooking polished rice comprises adding water and cooking the rice together with rice oil, which is obtained by preprocessing such as an enzyme inactivation process applied to fresh rice bran in a rice milling plant followed by compression process and physical refinement, and partly defatted rice bran remaining after obtaining the rice oil.

JP 2007 068523 A describes a rice bran composition which is roasted to remove various germs. The roasted rice bran is wrapped with a capsule or a soft capsule or compressed and hardened to afford a tablet-like supplement.

WO 2007/060947 A2 describes a health food composition containing protein, fat and sugar, wherein said ratio of saturated fatty acid and polyunsaturated fatty acid falls within a range of 1 : 1.8 to 1 : 4.

WO 2007/029631 A1 describes a composition for use in the amelioration of abnormal lipid metabolism comprising a rice bran protein.

KAHLON T.S. ET AL.: 'Cholesterol lowering effects of rice bran and rice bran oil fractions in hypercholesterolemic hamsters' CEREAL CHEM. vol. 69, no. 5, 1992, pages 485 - 489, (XP008128977) reports on a scientific study, in which Syrian golden hamsters were fed with different diets being divided into cholesterol-free diets and cholesterol diets. A full fat rice bran diet and combinations of defatted rice bran and cellulose are compared with respect to their effects on liver weight and amount of cholesterol and triglycerides in the plasma and the livers of the hamsters.

[Disclosure of the Invention]

[Technical Problem]

An object of the present invention is to provide a rice bran-like composition having better taste and smell and higher bioactivity by modifying rice bran.

[Technical Solution]

The present inventors found that a mixture in which defatted rice bran obtained by pressing rice bran to remove oil and rice bran oil obtained by refining pressed rice bran oil are mixed in a weight ratio of 85:15 to 60:40 has an effect of reducing or suppressing an increase in visceral fat (mesenteric fat); an effect of reducing or suppressing an increase in neutral fat in blood; and an effect of reducing or suppressing an increase in blood cholesterol, all the effects being superior to those of rice bran which is a raw material of the mixture. The present inventors accomplished the present invention based on these findings.

Specifically, the present invention provides the following use of a rice bran-like composition, and a food.

Item 1. A rice bran-like composition containing (1) defatted rice bran obtained by pressing rice bran to remove oil and (2) rice bran oil obtained by refining pressed rice bran oil, wherein a weight ratio of the defatted rice bran content to the rice bran oil content is 80:20 to 70:30 for use in reducing or suppressing an increase in visceral fat.

Item 2. The rice bran-like composition according to Claim i, wherein the fat content is 20 to 40 wt.%, the protein content is 10 to 17 wt.%, the carbohydrate content is 5 to 21 wt.%, the ash content is 7 to 13 wt.%, and the fiber content is 20 to 32 wt.%.

Item 3. The rice bran-like composition according to Claim 1 or Claim 2, wherein the wax content is not more than 1.1 wt.% and the free fatty acid content is 0.5 to 5 wt.%.

Item 4. A food containing the rice bran-like composition according to any one of items 1 to 3, for use in reducing or suppressing an increase in visceral fat.

Item 5. The food according to Claim 4.

The rice bran-like composition of the present invention is a mixture in which defatted rice bran obtained by pressing rice bran to remove oil is mixed with rice bran oil obtained by refining pressed rice bran oil such that the weight ratio of defatted rice bran content to rice bran oil content is 80:20 to 70:30. Blending the rice bran oil with defatted rice bran in the above-described compounding ratio produces an effect of suppressing the accumulation of visceral fat and reducing blood fat. This effect is superior to that of rice bran. Further, the taste and smell are also improved.

The amount of each component in a preferable rice bran-like composition of the present invention is in the range shown in Table 1. The above-mentioned composition ratio can be suitably adjusted so that the amount of each component will be in the range.

[Table 1]

|  | Preferable Rice Bran-like Composition |
| --- | --- |
| Moisture | 2 to 8 wt.% |
| Protein | 10 to 17 wt.% |
| Fat | 20 to 40 wt.%<br>(more preferably 30 to 40 wt.%) |
| Carbohydrate | 5 to 21 wt.%<br>(more preferably 10 to 16 wt.%) |
| Ash | 7 to 13 wt.% |
| Fiber | 20 to 32 wt.%<br>(more preferably 24 to 28 wt.%) |
| Wax | 1.1 wt.% or lower<br>(more preferably 0.6 wt.% or lower) |
| Free fatty acid | 0.5 to 5 wt.%<br>(more preferably 1.0 to 3.5 wt.%) |

The main components of rice bran per 100 g are as shown in Table 2 below. Note that the values of moisture, protein, fat, carbohydrate, fiber, and ash shown in Table 2 are in accordance with the Standard Tables of Food Composition in Japan, 4th revised edition.

[Table 2]

|  | Rice Bran |
| --- | --- |
| Moisture | 13.5 g |
| Protein | 13.2 g |
| Fat | 18.3 g |
| Carbohydrate | 38.3 g |
| Ash | 8.9 g |
| Fiber | 7.8 g |

The rice bran-like composition of the present invention is a mixture of defatted rice bran derived from rice bran and rice bran oil from rice bran. However, because gum, wax, and the like are removed when rice bran oil is refined from the pressed oil, the rice bran-like composition of the present invention is different from rice bran. As shown in Tables 1 and 2, a particularly preferable rice bran-like composition greatly differs from rice bran in terms of the amount of each component. The measurement of oryzanol by high performance liquid chromatography indicated that the oryzanol content was about the same in unprocessed rice bran and in the rice bran-like composition of the present invention, specifically, 451 mg/100 g and 440 mg/100 g, respectively.

The moisture content, protein content, fat content, carbohydrate content, fiber content, ash content, energy, wax content, and free fatty acid content of the present invention are the amounts obtained by the following measurement methods.

<Method for Measuring Moisture Content>: Normal Pressure, Heat-Drying Method

A test sample is precisely located in an aluminum container whose mass is constant (mass: Xg), and the total mass of the container and the test sample (mass: Yg) is measured. Then, the test sample is dried for 3 hours at 135°C using a forced circulation hot air dryer. After the process, the test sample is allowed to cool in a desiccator, and the total mass of the container and the test sample (mass: Zg) is accurately measured.

```
Calculation Formula
Moisture content (g/100 g)=(Y-Z)/ [(Y-X)*100]
```

<Method for Measuring the Protein Content>: Kjeldahl Method.

Kjeldahl test is performed according to conventional procedures.

Used Equipment: KJELTEC AUTO SAMPLER SYSTEM ANALYZER)
Nitrogen/protein conversion factor: 5.96

<Method for Measuring Fat Content>: Acid Digestion Method or Diethyl Ether Extraction Method

Acid Digestion Method

The test sample is accurately weighed out (Xg), dispersed into a mixture of ethanol and hydrochloric acid, and then heated in a hot-water bath for 40 minutes. Thereafter, extraction using diethyl ether and petroleum ether is repeated three times. Then, all the ether layers are transferred into a separating funnel and sufficiently washed until the ether layers indicate no acidity. Thereafter, the ether is distilled off, and the residue is dried at 105°C for one hour. Then, the mass of remaining fat is measured.

Diethyl ether extraction method

A sample (10 g) was weighed out into a filter paper thimble. While being loosely covered with cotton, the sample was placed into a Soxhlet extractor. About 100 ml of diethyl ether was added to an extraction flask (weight known), and set in the Soxhlet extractor. Heat extraction was then performed in a water bath for 5 hours. The extraction rate was adjusted so that the solvent circulated about 12 to 13 times per hour. After completion of the extraction, the extraction flask was mounted to a rotary evaporator to completely evaporate the ethyl ether. The extraction flask was allowed to cool to room temperature and then weighed.

$$\text{Fat (\%)} = A/B \times 100$$

A: amount of extracted fat (g)
B: amount of sample (g)

<Method for Measuring the Carbohydrate Content>

The following formula is based on a formula according to Nutrition Labeling Standards (Ministry of Health, Labor and Welfare Notification No. 176, 2003).

$$100-(\text{moisture} + \text{protein} + \text{fat} + \text{ash} + \text{dietary fiber})$$

<Method for Measuring Dietary Fiber Content>: Enzyme-Weight Method

Two sets (15 to about 20 mg each) of the test samples are weighed out, and the following process is performed on each. After defatting a test sample with petroleum ether, phosphate buffer (pH 6.0) and Termamyl (Novozymes 120L) are added thereto, and the resulting mixture is then heated with shaking for 30 minutes in a boiling-water bath. After cooling, sodium hydroxide is added to the mixture to adjust the pH thereof to 7.5. Then, phosphate buffer and proteolytic enzyme (Sigma P-5380) are added to the mixture, which is then heated with shaking at 60°C for 30 minutes. After cooling, hydrochloric acid is added to the sample to adjust the pH thereof to 4.3. Then, amyloglucosidase (Sigma A-9913) is added thereto, and the resulting mixture is then heated with shaking at 60°C for 30 minutes. After cooling, the mixture is filtered by suction through a glass filter, and the residue is washed using ethanol and acetone. Then the residue is dried at 105°C for half a day. The mass of each resulting residue ($R_1$, $R_2$) is precisely weighed. One test sample is converted into ash under the conditions of 525°C for 5 hours, and the ash content thereof is measured. The other is measured for the protein content by the Kjeldahl method (nitrogen/protein conversion factor: 6.25). The obtained protein content and ash content are applied to the following calculation formulae to calculate the dietary fiber content. Calculation Formulae

[Math. 1]

$$\text{Dietary fiber (g/100g)} = \frac{R \times (1 - \frac{P+A}{100}) - B}{S} \times 100 \times \frac{100-F}{100}$$

R:  weight of the residues [average value, $(R_1+R_2)/2$, mg]
P:  protein (%) in the residue
A:  ash (%) in the residue
S:  amount of defatted sample collection (average value, mg)
F:  weight reduction by the defatting process (%)
B:  blank (mg)

[Math. 2]

$$B\ (mg) = r \times (1 - \frac{p+a}{100})$$

r:  weight of blank residue (average value, mg)
p:  protein in blank residue (%)
a:  ash in blank residue (%)

<Method for Measuring the Ash Content>: Direct Ashing Method

A porcelain container is used to contain the test sample whose mass has been measured and is then heated at 550°C until a constant mass is obtained. Then the mass after cooling is precisely weighed.

<Method for Measuring the Amount of Energy>

A conversion factor for each nutrient component based on the calculation formula according to Nutrition Labeling Standards (Ministry of Health, Labor and Welfare Notification No. 176, 2003) is as follows: 4 for protein, 9 for fat, 4 for carbohydrate, and 2 for dietary fiber.

<Method for Measuring Wax Content>

After fat was extracted from the test sample using petroleum ether, the petroleum ether was distilled off. The resulting sample (20 g) was placed in a 200 ml-conical flask. 120 ml of MEK (methyl ethyl ketone) was added thereto, and then dissolved by heating. The resultant was allowed to cool or cooled in water to 5 to 6°C. Subsequently, suction filtration was performed using filter paper of known weight (No.2, 7 cm or 9 cm). After the filter paper with the residue was air-dried, the amount of the residue was weighed.

$$Wax\ (wt.\%) = A/B \times C$$

A:  amount of residue (g)
B:  amount of sample (g)
C:  oil content (wt.%)

<Method for Measuring Free Fatty Acid Content>

100 g of the test sample is precisely weighed, and the fat is extracted using petroleum ether. The petroleum ether is distilled off, and the remaining fat is measured using capillary chromatography, LC-MS, etc., according to conventional procedures. The measurement objects are palmitic acid, oleic acid, and linoleic acid, and the total of each value is regarded as the free fatty acid content.

Hereinbelow, a method of producing the rice bran-like composition of the present invention will be described. Rice bran used as raw material for production comprises the pericarp, seed coat, perisperm, aleurone layer, etc. of brown rice, and usually accounts for about 8 to about 10 wt.% of brown rice. Rice bran is obtained as a by-product when brown rice is polished to produce white rice. Accordingly, the present invention can use the rice bran. The rice bran (raw rice bran) obtained after polishing usually contains 18 to 20 wt.% of fat. The lipase-mediated oxidation of oil in raw rice bran proceeds quickly, and it is thus preferable to deactivate lipase in raw rice bran. The process of deactivating lipase in raw rice bran is known. Generally, raw rice bran is roasted by heating at about 70°C to about 130°C. Such a known process of deactivation of lipase is also applicable to the present invention. Further, examples of a heating means include a cooking device, dry extruder, wet extruder, and steam treatment device. Note that heat-treated rice bran is commercially available. Such commercially available rice bran can also be used in the present invention. Further, predrying in which raw rice bran is dried to prepare dry rice bran having about 5 to about 10 wt.% of moisture content may be carried out before roasting by heating.

The heat-treated rice bran is pressed, thereby producing pressed oil and defatted rice bran, which is the pressed residue. The defatted rice bran produced by pressing is different from defatted rice bran produced by solvent extraction (particularly, nonpolar or low-polar organic solvent extraction). Organic solvent extraction has a higher oil yield than oil pressing. Accordingly, the degree of defatting of defatted rice bran (the defatted rice bran of the present invention) obtained by pressing rice bran is lower than that of defatted rice bran obtained by organic solvent extraction. Normally, the solvent extraction method is employed for the production of rice bran oil; however, the pressing method is employed for the rice bran oil of the present invention. The pressing method is known, in which, for example, rice bran roasted by heating to about 100 to about 115°C is pressed by a low-temperature, continuous pressing machine (for example, Miracle Chamber sold by Techno Sigma Co., Ltd.). The press method is not limited to the above method: other known press methods can be applied to the present invention. The degree of pressing is adjusted such that the fat in defatted rice bran after pressing is 5 to 15 wt.%, preferably 5 to 14 wt.%, more preferably 5 to 12 wt.%.

The defatted rice bran obtained by pressing can be utilized *as is* in the food and the rice bran-like composition of the present invention. It is preferably processed into powder, which is easy to handle, by grinding and grading. For example, because the shape of defatted rice bran immediately after low-temperature pressing is not uniform, clumps of defatted rice bran are broken down using a cracking machine (e.g., a detacher) to obtain a uniform particle size. After the clumps are broken down, the defatted rice bran is made into powdery defatted rice bran by using a grinding mill. Examples of grinding mills include a hammer mill, a pin mill, a roller mill, a stone mill, a disk mill, etc. Further, the defatted rice bran obtained by pressing may be roasted at about 100 to about 120°C to provide improved stability before breaking down the clumps of defatted rice bran.

An average particle diameter of preferable defatted rice bran is 50 to 200 $\mu$m, more preferably 50 to 100 $\mu$m. Further, the fat content in defatted rice bran is preferably 5 to 15 wt.%, and more preferably 5 to 10 wt.%.

The pressed oil (crude oil of rice bran oil) obtained by pressing is refined into rice bran oil. The crude oil obtained by pressing usually contains about 3 wt.% of wax and about 30 wt.% of free fatty acid content. However, the rice bran oil used in the present invention usually contains a wax content and the free fatty acid content of not more than 0.5 wt.% and not more than 5 wt.%, respectively, preferably not more than 0.1 wt.% and not more than 1 wt.%, respectively. The method of refining rice bran oil from crude oil is known. For example, rice bran oil is refined to a degree required for edible use by performing the following processes on crude oil: removing foreign substances (foreign substance removal), removing gum (degumming), removing acid (deacidification), removing wax (dewaxing), decoloring, and wintering in this stated order, or by combining these processes in an appropriate order, with additional processes as needed. A known method of producing rice bran oil from crude oil can be employed in the present invention.

The main steps of refining crude oil will be described hereinbelow. Refinement of crude oil usually proceeds in the following order: a degumming process, dewaxing process, decoloring process, deacidifying process, and wintering process.

The degumming process is a process in which, for example, water and acids (such as phosphoric acid) are added to the oil to be treated to form insoluble matter (gum), and the insoluble matter is removed by means of centrifugal separators and the like.

The dewaxing process is a process in which, for example, the oil to be treated is left to stand (usually for a few days to about one week) at low temperatures (usually 10 to 30°C) to precipitate wax, and the precipitated wax is removed by means of filtration and the like.

The decoloring process is a process in which, for example, the oil to be treated is mixed with an adsorbent such as activated acid clay, which is dispersed therein to absorb coloring matter, and the adsorbent is then removed by means of filtering devices and the like such as a filter press.

For the deacidifying process, there are mainly two types of methods that are generally used: alkali deacidification and deacidification by distillation. Alkali deacidification is a method in which, for example, the oil to be treated is blended with alkali (such as sodium hydroxide), thereby turning acids such as free fatty acids into insoluble saponified matter, and this insoluble matter is then removed by means of centrifugal separators and the like. Further, deacidification by distillation is a method in which, for example, steam is injected into the oil to be treated under high temperature vacuum conditions, thereby removing acids such as free fatty acids by evaporation.

The wintering process is a process in which, for example, the oil to be treated is left to stand (usually for a few days) at low temperatures (usually 0 to 10°C) to precipitate wax that could not be removed in the dewaxing process; this wax is then removed by means of filtration and the like.

Rice bran oil is mixed with the aforementioned defatted rice bran in a weight ratio of 80:20 to 70:30 (defatted rice bran : rice bran oil), thereby the rice bran-like composition of the present invention is obtained. The mixing procedure is not particularly limited.

Also, other additional materials may be added to the rice bran-like composition. Furthermore, the rice bran-like composition may be blended, as needed, with excipients, diluents, carriers, and like components that are available in the fields of oral compositions and foods so as to be formed into powders, granules, pellets, flakes, blocks, and the like by known methods. Accordingly, the rice bran-like composition is usable as an oral composition and a food, and thus the present invention may include such embodiments. Preferable forms are powders and granules.

Further, the rice bran-like composition is usable as a food additive. The bioactivity, nutrients, and flavor of the rice bran-like composition can be added to known foods as the rice bran-like composition is added to such foods.

The food and the oral composition of the present invention contain the rice bran-like composition of the present invention, or defatted rice bran obtained by pressing rice bran to remove oil and rice bran oil obtained by refining the pressed rice bran oil such that the weight ratio of defatted rice bran content to rice bran oil content is 80:20 to 70:30. The food of the present invention can be prepared by combining the rice bran-like composition, defatted rice bran, and rice bran oil with carriers and the like according to a usual method. The food can be used in various forms as follows according to the purpose of its use and the like: liquid formulations and solid formulations such as pills, granules, fine granules, powders, and tablets; or other formulations containing the aforementioned liquid or solid formations such as capsules, troches, emulsions, gels, and the like. Preferable forms are pills, granules, fine granules, and tablets. Examples of carriers include excipients, diluents, and the like. Further, the food and the oral composition of the present invention may contain various additives such as perfume as need.

Examples of carriers and additives include the following materials: excipients such as sugar alcohols (maltitol, xylitol, sorbitol, erythritol, etc.), lactose, sucrose, sodium chloride, glucose, starch, and carbonates (calcium carbonate, etc.), kaolin, microcrystalline cellulose, silicic acid, methyl cellulose, glycerin, sodium alginate, gum arabic, talc, phosphates (calcium monohydrogen phosphate, calcium hydrogen phosphate, sodium hydrogen phosphate, dibasic potassium phosphate, potassium dihydrogen phosphate, calcium dihydrogen phosphate, sodium dihydrogen phosphate, etc.), calcium sulfate, calcium lactate, cacao oil, etc.; viscosity modifiers such as simple syrup, glucose solution, starch solution, gelatin solution, etc.; binders such as polyvinyl alcohol, polyvinyl ether, polyvinylpyrrolidone, cross-polyvinylpyrrolidone, hydroxypropyl cellulose, low-substitution hydroxypropyl cellulose, hydroxypropyl methylcellulose, hydroxyethyl cellulose, carboxy vinyl polymer, microcrystalline cellulose, powdered cellulose, microcrystalline cellulose and carboxymethylcellulose sodium, carboxymethyl cellulose, shellac, methyl cellulose, ethyl cellulose, potassium phosphate, powdered Acacia, pullulan, pectin, dextrin, corn starch, alpha starch, hydroxypropyl starch, gelatin, xanthan gum, carrageenan, tragacanth, powdered tragacanth, macrogol, etc.; disintegrants such as dry starch, sodium alginate, powdered agar, powdered laminaran, sodium bicarbonate, calcium carbonate, polyoxyethylene sorbitan fatty acid esters, sodium lauryl sulfate, stearic acid monoglyceride, starch, lactose, etc.; disintegration suppressing agents such as sucrose, stearic acid, cacao butter, hydrogenated oil, etc.; absorption promoting agents such as quarternary ammonium salt, sodium lauryl sulfate, etc.; adsorbents such as starch, lactose, kaolin, bentonite, colloidal silicic acid, etc.; lubricants such as refined talc, stearic acid salt, boracic acid powder, polyethylene glycols, etc.; emulsifiers such as sucrose fatty acid ester, sorbitan fatty acid ester, enzyme-treated lecithin, enzyme-decomposed lecithin, saponin, etc.; antioxidant agents such as ascorbic acid, tocopherol, etc.; acidulants such as lactic acid, citrate, gluconic acid, glutamic acid, etc.; reinforcing agents such as vitamins, amino acids, lactic acid salt, citric salt, gluconic acid salt, etc.; fluidizing agents such as silicon dioxide, etc.; sweeteners such as sucralose, acesulfame potassium, aspartame, glycyrrhizin, etc.; fragrances such as mint oil, eucalyptus oil, cinnamon oil, fennel oil, clove oil, orange oil, lemon oil, rose oil, fruit flavor, mint flavor, peppermint powder, dl-menthol, 1-menthol, etc.; oligosaccharides such as lactulose, raffinose, lactosucrose, etc.; and manufacturing agents such as sodium acetate, etc.

Further, according to the needs, solid formulations such as pills and the like may be formed into tablets coated with usual coating materials to provide, for example, sugar-coated tablets, gelatin-coated tablets, enteric-coated tablets, film-coated tablets, double-layer tablets, multi-layer tablets, etc. Capsule formulations are prepared by mixing the rice bran-like composition with various additives or various carriers as needed, and filling the mixture into hard gelatin capsules, soft gelatin capsules, and the like. Liquid preparations may be aqueous or oily suspensions, solutions, syrups, or elixirs, and can be prepared using regular carriers, additives, and the like according to conventional procedures.

The content of defatted rice bran in the food of the present invention is usually 5 to 70 wt.%, preferably 25 to 55 wt.%. The content of rice bran oil is usually 1 to 20 wt.%, preferably 5 to 15 wt.%.

Examples of foods of the present invention include supplement tablets, chewable tablets, bread, cookies, rice snacks, cake, cereal, noodles, cereal bars, soup, *furikake,* seasonings, granules, fine granules, powders, powdered drinks, salad dressings, additives for rice, precooked food (retort food), etc. Further, the food of the present invention can also be treated as a food for specific purposes, such as a health food, functional food, food for specified health uses, food with nutrient function claims, food for diseased individuals, etc.

[Advantageous Effects]

The rice bran-like composition of the present invention has an effect of reducing or suppressing an increase in visceral fat (mesenteric fat); an effect of reducing or suppressing an increase in neutral fat in blood; and an effect of reducing or suppressing an increase in blood cholesterol. Further, because the rice bran-like composition of the present invention has an effect of reducing or suppressing an increase in the neutral fat content in the VLDL fraction, the rice bran-like composition also has an effect of suppressing arteriosclerosis.

Accordingly, the rice bran-like composition and the food of the present invention can be a food for people who tend to eat foods high in fat; a food for people who are concerned about visceral fat; a food for people who are concerned about neutral fat; a food for people who are concerned about cholesterol; a food for overweight people; a food for people whose blood neutral fat level tends to be high; a food for people who often eat out; a food for people who eat large amounts of meat and dairy products; a food for people who eat large amounts of processed foods; and a food for improving the diet of those people listed above.

Further, results of test examples show that the following effects can be expected from the rice bran-like composition and the food of the present invention: an effect of preventing ischemic heart disease based on the results regarding the neutral fat content in blood and the neutral fat content in each fraction; an effect of increasing or suppressing a decrease in adiponectin secretion based on the result regarding the amount of body fat; an effect of improving insulin resistance based on the result regarding the amount of body fat; and an effect of preventing diabetics based on the result regarding the amount of body fat.

[Best Mode for Carrying out the Invention]

Hereinafter, the present invention will be explained in detail with reference to the examples; however, the present invention is not limited thereto.

EXAMPLES

<Example 1: Production of rice bran-like composition>

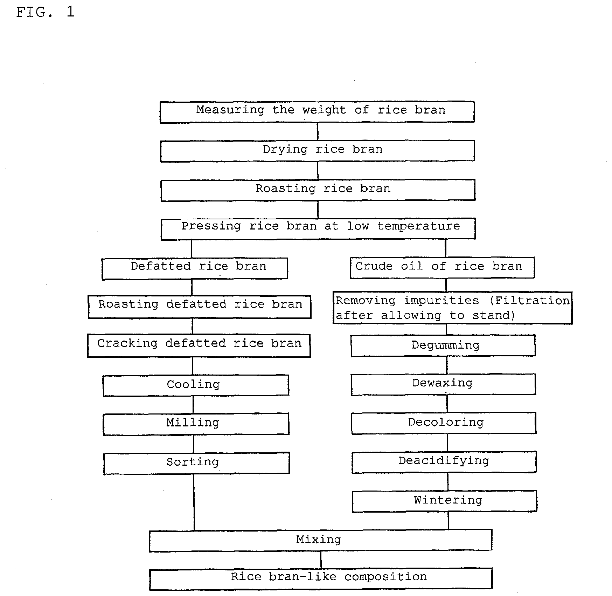

A rice bran-like composition was prepared according to the processes shown in FIG. 1.

1. Low temperature press

Immediately after polishing, crude rice bran (moisture content: 14 to 15 wt.%) was flash-dried by hot air to obtain dried rice bran having a moisture content of 7 to 8 wt.%. The dried rice bran was roasted by heating at 105 to 110°C for 0.5 hours, thereby obtaining a heat-roasted rice bran having a moisture content of 4 to 5 wt.%. The heat-roasted rice bran was pressed at low temperature at a capacity of 200 kg/h using a low-temperature press (Miracle Chamber, product of Techno Sigma, Co., Ltd.), thereby obtaining crude rice bran oil at a rate of 25 kg/h and defatted rice bran at a rate of 170 kg/h. The resulting defatted rice bran had a moisture content of 0.6 wt.% and a fat content of 8 wt.%. The wax content and the fat content were measured according to the following method.

Wax

After fat was extracted from the test sample using petroleum ether, the petroleum ether was distilled off. The resulting sample (20 g) was placed in a 200 ml-conical flask. 120 ml of MEK (methyl ethyl ketone) was added thereto, and then dissolved by heating. The resultant was allowed to cool or cooled in water to 5 to 6°C. Subsequently, suction filtration was performed using filter paper of known weight (No.2, 7 cm or 9 cm). After the filter paper with the residue was air-dried, the amount of the residue was weighed.

$$Wax\ (wt.\%) = A/B \times C$$

A: amount of residue (g)
B: amount of collected sample (g)
C: oil content (wt.%)

Fat: Diethyl ether extraction method

A sample (10 g) was weighed out into a filter paper thimble. While being loosely covered with cotton, the sample was placed into a Soxhlet extractor. About 100 ml of diethyl ether was added to an extraction flask (weight known), and set in the Soxhlet extractor. Heat extraction was then performed in a water bath for 5 hours. The extraction rate was adjusted so that the solvent circulated about 12 to 13 times per hour. After completion of the extraction, the extraction flask was mounted to a rotary evaporator to completely evaporate the ethyl ether. The extraction flask was allowed to cool to room temperature and then weighed.

$$Fat\ (\%) = A/B \times 100$$

A: amount of extracted fat (g)
B: amount of collected sample (g)

## 2. Powderization of defatted rice bran

The defatted rice bran was broken down using a detacher, and then milled using a roll mill. The resulting powders were screened to obtain powdery defatted rice bran having an average particle diameter of about 150 $\mu$m.

## 3. Process of producing rice bran oil by refining crude oil

The crude oil obtained from the low temperature press was refined according to the following processes: a degumming process, dewaxing process, decoloring process, deacidifying process, and wintering process. The processes were performed in this order.

First, foreign substances were removed from the crude oil. Water and acids (phosphoric acid etc.) were added to the resulting oil to obtain gum as insoluble matter. The gum was then removed using a centrifugal separator (degumming process).

The resulting degummed oil was allowed to stand at low temperature (20 to 30°C) for 3 days to precipitate wax. The precipitated wax was then removed by filtration (dewaxing process).

The resulting dewaxed oil was mixed with an adsorbent (activated acid clay), and dispersed by stirring to adsorb coloring substances. The adsorbent was then separated by filtration using a filter press (decoloring process).

Next, steam was injected into the decolored oil under high temperature vacuum conditions to evaporate and remove acids such as free fatty acids (deacidifying process).

The deacidified oil was allowed to stand at low temperature (0° to 5°C) for 3 days. The precipitated wax was then removed by filtration, thereby obtaining rice bran oil (wintering process).

The wax content and the free fatty acid content of the resulting rice bran oil were measured. The measurement revealed that both values were 1 wt.% or less.

## 4. Step of mixing rice bran oil with powdery defatted rice bran

The resulting powdery defatted rice bran and rice bran oil were fed into a paddle mixer at a weight ratio of 3:1 and mixed to obtain a rice bran-like composition.

Test Example 1: Body fat content, cholesterol content, neutral fat content of high-fat diet rats

Using rats, the effect of ingesting a high-fat diet, and the effect of the rice bran composition of the present invention thereon were examined.

Male 4-week old SD rats (microorganism grade; SPF/VAF, produced by Charles River, Japan, Inc.) were divided into groups (six rats per group) and subjected to the test.

The rats were housed in a stainless-steel, six-in-row cage under the following conditions: temperature: 24±1°C, humidity: 55±5%, lighting cycle: 12 hours from 8h to 20h.

The compositions of the standard diet (Cont.), high-fat polished white rice diet (WR), high-fat brown rice diet (BR), and high-fat test diet (diet containing high-fat polished white rice and the rice bran-like composition obtained in Example 1) (WR+RP) are shown in Table 3. The standard diet contains soybean oil without containing lard. The other diets are high-fat diets each containing lard (pork fatback) without containing soybean oil. In these respective diets, $\alpha$-cornstarch, which is the main carbohydrate of the standard diet, is replaced by $\alpha$-polished white rice powder, $\alpha$-brown rice powder, or $\alpha$-polished white rice powder and a rice bran-like composition (weight ratio= 9:1). The WR+RP diet contains 10 wt.% of rice bran-like composition because brown rice contains rice bran in an amount of about 10 wt.%.

Table 3

| Wt.% | Standard diet (Cont.) | High-fat polished white rice diet (WR) | High-fat brown rice diet (BR) | High-fat test diet (WR+RP) |
|---|---|---|---|---|
| Casein | 20.0 | 20.0 | 20.0 | 20.0 |

(continued)

| Wt.% | Standard diet (Cont.) | High-fat polished white rice diet (WR) | High-fat brown rice diet (BR) | High-fat test diet (WR+RP) |
|---|---|---|---|---|
| L-cystine | 0.3 | 0.3 | 0.3 | 0.3 |
| Soy bean oil | 7.0 | 0 | 0 | 0 |
| Lard | 0 | 15.0 | 15.0 | 15.0 |
| Cellulose | 5.0 | 5.0 | 5.0 | 5.0 |
| AIN 93G mineral mixture | 3.5 | 3.5 | 3.5 | 3.5 |
| AIN 94G mineral mixture | 1.0 | 1.0 | 1.0 | 1.0 |
| Choline bitartrate | 0.25 | 0.25 | 0.25 | 0.25 |
| Tert-butylhydroquinone | 0.0014 | 0.003 | 0.003 | 0.003 |
| $\alpha$-Cornstarch | 62.9486 | 0 | 0 | 0 |
| $\alpha$-Polished white rice powder | 0 | 54.9470 | 0 | 49.4523 |
| $\alpha$-Brown rice powder | 0 | 0 | 54.9470 | 0 |
| Rice bran-like composition | 0 | 0 | 0 | 5.4947 |
| Total | 100 | 100 | 100 | 100 |

Table 4 below shows the nutrient analysis values (moisture content, crude protein content, crude fat content, carbohydrate content, and crude ash content) of the diets.

[Table 4]

| Wt.% | Standard diet (Cont.) | High-fat polished white rice diet (WR) | High-fat brown rice diet (BR) | High-fat test diet (WR+RP) | Measurement method |
|---|---|---|---|---|---|
| Moisture | 10.8 | 4.0 | 3.8 | 3.7 | Normal pressure heat-drying method |
| Crude protein | 17.9 | 21.0 | 21.1 | 21.0 | Kjeldahl method |
| Crude fat | 6.9 | 14.7 | 16.3 | 16.4 | Diethyl ether extraction method |
| Carbohydrate | 61.9 | 57.8 | 55.7 | 55.9 | Carbohydrate measuring method |
| Crude ash | 2.5 | 2.5 | 3.1 | 3.0 | Direct ashing method |

Each rat was fed the standard diet for one week, and then fed their respective diet for 4 weeks. The rats had free access to food and purified water.

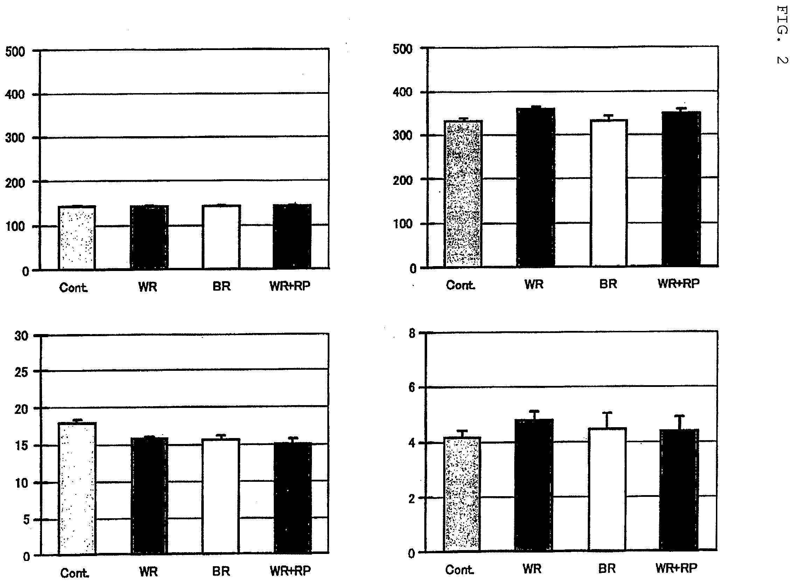

After 4 weeks of feeding, the following items were measured. The results are shown in FIGs. 2 to 5.

Initial weight (FIG. 2, upper left), final weight (FIG. 2, upper right), diet intake (FIG. 2, lower left), total fat rate (FIG. 2, lower right);

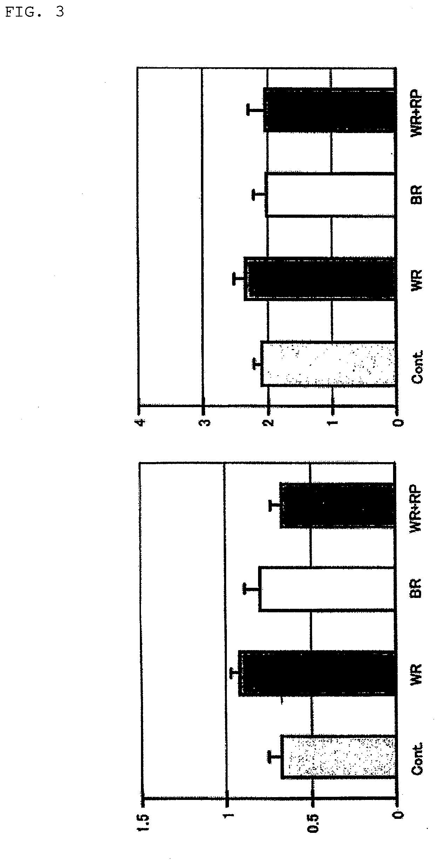

body fat content (mesentery (FIG. 3, left), posterior abdominal wall (FIG. 3, right));

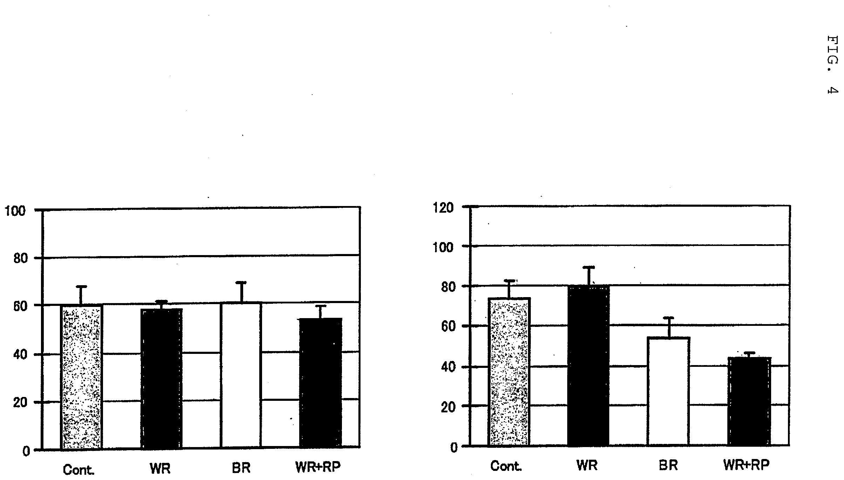

total cholesterol content in blood (FIG. 4, left), neutral fat content in blood (FIG. 4 right);

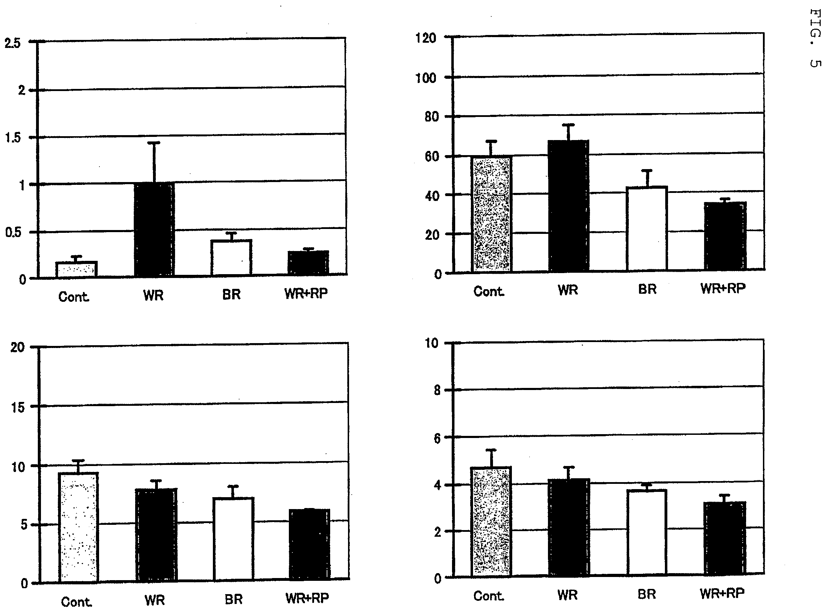

neutral fat content in the CM (chylomicron) fraction (FIG. 5, upper left), neutral fat content in the VLDL fraction (FIG. 5, upper right), neutral fat content in the LDL fraction (FIG. 5, lower left), neutral fat content in the HDL fraction (FIG. 5, lower right).

<Amount of body fat>

The high-fat test diet group showed a large decrease in mesenteric fat (visceral fat) compared to the high-fat polished white rice diet group. The degree of the decrease was noticeably larger than that of the high-fat brown rice diet group. The mesenteric fat content of the high-fat brown rice diet group was larger than that of the standard diet group, whereas the mesenteric fat content of the high-fat test diet group was almost the same as that of the standard diet group to which a high-fat diet was not fed. The high-fat test diet group did not show a significant decrease in body fat except for mesenteric fat. The results reveal that the fat decrease was specific to mesenteric fat.

<Total cholesterol content in blood>

The standard diet group, high-fat polished white rice diet group, and high-fat brown rice diet group showed almost the same level of total cholesterol content in blood; however, the high-fat test diet group showed a significant decrease.

<Neutral fat content in blood>

The high-fat polished white rice diet group showed an increase compared to the standard diet group. The high-fat brown rice diet group showed a decrease compared to the standard diet group; however, the high-fat test diet group showed a further decrease compared to the high-fat brown rice diet group.

<Neutral fat content in each fractionation>

The neutral fat content in each of the CM fraction and VDLD fraction showed the same tendency as the aforementioned neutral fat content in blood. In each fraction, the high-fat test diet group showed a lower level than the high-fat brown rice diet group.
The results reveal that the rice bran-like composition and the food of the present invention have the following effects. - the effect of preventing or improving lipid abnormalities (based on the results regarding the total cholesterol content in blood, neutral fat content in blood, and neutral fat content in each fraction).

- the effect of suppressing intestinal absorption of fat (particularly neutral fat) (based on the results regarding the neutral fat content in blood and neutral fat content in each fraction).
- the effect of preventing arteriosclerosis (based on the results regarding the neutral fat content in blood and neutral fat content in each fraction).
- the effect of preventing or ameliorating metabolic syndrome (based on the results regarding the amount of body fat).

<Formulation examples>

The following foods were prepared according to known methods using the materials below. The powdery defatted rice bran and the rice bran oil used in the formulation examples were prepared in the same manner as in Example 1. "%" indicates "percentage by weight".

Brown rice *furikake*

| | |
|---|---|
| Rice bran-like composition | 40% |
| (Defatted rice bran | 30%) |
| (Rice bran oil | 10%) |
| Black sesame paste (fat: 60%) | 20% |
| Red perilla | 35% |
| Monosodium glutamate | 5% |
| Total | 100% |

*Takikomi* brown rice stock

| | |
|---|---|
| Defatted rice bran | 40% |
| Rice bran oil | 15% |
| Cornstarch | 10% |

(continued)

| Salt | 15% |
|---|---|
| Monosodium glutamate | 8% |
| Scallop extract | 3% |
| Crab extract | 7% |
| Powdered soy sauce | 2% |
| Total | 100% |

### Powdered soup

| Rice bran-like composition | 40% |
|---|---|
| (Defatted rice bran | 30%) |
| (Rice bran oil | 10%) |
| Sweet corn powder | 25% |
| White pepper | 0.2% |
| Salt | 6% |
| Granulated sugar | 15% |
| Yeast extract | 6% |
| Monosodium glutamate | 6% |
| Pregelatinized starch | 1.8% |
| Total | 100% |

### Powdered brown rice beverage (not part of the invention)

| Rice bran-like composition | 65% |
|---|---|
| (Defatted rice bran | 55%) |
| (Rice bran oil | 10%) |
| Soybean powder | 12% |
| Black sesame paste (fat: 60%) | 15% |
| Powdered brown sugar | 8% |
| Total | 100% |

### Brown rice dried noodles

| Defatted rice bran | 7.5% |
|---|---|
| Rice bran oil | 2.5% |
| Wheat flour | 80% |
| Salt | 10% |
| Total | 100% |

### Cereal bar

| Defatted rice bran | 20% |
|---|---|
| Rice bran oil | 8% |
| Soybean flour | 2% |
| Cornflakes | 5% |
| Granola | 15% |
| Starch syrup | 45% |
| Mix nuts | 5% |
| Total | 100% |

#### Supplement (granule)

| | |
|---|---|
| Rice bran-like composition | 40% |
| (Defatted rice bran | 30%) |
| (Rice bran oil | 10%) |
| Powder maltitol | 30% |
| Cocoa powder | 29% |
| Powder perfume | 1% |
| Total | 100% |

#### Supplement (tablet)

| | |
|---|---|
| Rice bran-like composition | 40% |
| (Defatted rice bran | 30%) |
| (Rice bran oil | 10%) |
| Dextrin | 30% |
| Kale powder | 30% |
| Total | 100% |

#### Cake mix powder

| | |
|---|---|
| Rice bran-like composition | 8% |
| (Defatted rice bran | 6%) |
| (Rice bran oil | 2%) |
| Wheat flour | 60% |
| Sugar | 20% |
| Soybean flour | 7% |
| Baking powder | 5% |
| Total | 100% |

Brief Description of the Drawings

FIG. 1 is a flow chart showing one embodiment of the production process of the defatted rice bran and rice bran oil in the rice bran-like composition of the invention.

FIG. 2 shows the initial rat weight (g) (upper left), the final rat weight (g/28 days) (upper right), the dietary intake (g/day) (lower left), and the percentage of total fat (%) (lower right) measured in Test Example 1.

FIG. 3 shows the fat amount of each site relative to the final rat weight measured in Test Example 1. Specifically, FIG. 3 shows the amount of mesenteric fat (visceral fat) (%) (left), and the amount of posterior abdominal wall fat (%) (right).

FIG. 4 shows the total cholesterol content in blood (mg/dl) (left) and the neutral fat content (mg/dl) (right) measured in Test Example 1.

FIG. 5 shows the neutral fat content of each fraction measured in Test Example 1. Specifically, FIG. 5 shows the neutral fat (mg/dl) in the CM fraction (upper left), the neutral fat (mg/dl) in the VLDL fraction (upper right), the neutral fat (mg/dl) in the LDL fraction (lower left), and the neutral fat (mg/dl) in the HDL fraction (lower right).

**Claims**

1. A rice bran-like composition for use in reducing or suppressing an increase in visceral fat, the rice bran-like composition contains (1) defatted rice bran obtained by pressing rice bran to remove oil and (2) rice bran oil obtained by refining pressed rice bran oil, wherein a weight ratio of the defatted rice bran content to the refined rice bran oil content is 80:20 to 70:30.

**2.** The rice bran-like composition according to Claim 1, wherein the fat content is 20 to 40 wt.%, the protein content is 10 to 17 wt.%, the carbohydrate content is 5 to 21 wt.%, the ash content is 7 to 13 wt.%, and the fiber content is 20 to 32 wt.%.

**3.** The rice bran-like composition according to Claim 1 or Claim 2, wherein the wax content is not more than 1.1 wt.% and the free fatty acid content is 0.5 to 5 wt.%.

**4.** A food for use in reducing or suppressing an increase in visceral fat, wherein the food contains the rice bran-like composition according to anyone of Claims 1 to 3.

**Patentansprüche**

**1.** Reiskleieartige Zusammensetzung für die Verwendung bei der Verringerung oder der Unterdrückung einer Zunahme im Viszeralfett, wobei die reiskleieartige Zusammensetzung (1) entfettete Reiskleie, die durch Pressen von Reiskleie, um Öl zu entfernen, erhalten wird, und (2) Reiskleieöl, das durch Raffination von gepresstem Reiskleieöl erhalten wird, enthält, wobei ein Gewichtsverhältnis des Gehalts an entfetteter Reiskleie zu dem Gehalt an raffiniertem Reiskleieöl 80:20 bis 70:30 ist.

**2.** Reiskleieartige Zusammensetzung nach Anspruch 1, wobei der Fettgehalt 20 bis 40 Gew.-% ist, der Proteingehalt 10 bis 17 Gew.-% ist, der Kohlenhydratgehalt 5 bis 21 Gew.-% ist, der Aschegehalt 7 bis 13 Gew.-% ist und der Fasergehalt 20 bis 32 Gew.-% ist.

**3.** Reiskleieartige Zusammensetzung nach Anspruch 1 oder Anspruch 2, wobei der Wachsgehalt nicht mehr als 1,1 Gew.-% ist, und der Gehalt an freien Fettsäuren 0,5 bis 5 Gew.-% ist.

**4.** Lebensmittel für die Verwendung bei der Verringerung oder Unterdrückung einer Zunahme im Viszeralfett, wobei das Lebensmittel die reiskleieartige Zusammensetzung nach einem der Ansprüche 1 bis 3 enthält.

**Revendications**

**1.** Composition analogue au son de riz pour une utilisation dans la réduction ou la suppression d'une augmentation de la graisse viscérale, la composition analogue au son de riz contenant (1) du son de riz dégraissé obtenu en pressant du son de riz pour éliminer l'huile et (2) de l'huile de son de riz obtenue en raffinant l'huile de son de riz pressée, dans laquelle un rapport pondéral de la teneur en son de riz dégraissé sur la teneur en huile de son de riz raffinée est de 80/20 à 70/30.

**2.** Composition analogue au son de riz selon la revendication 1, dans laquelle la teneur en graisses est de 20 à 40 % en poids, la teneur en protéines est de 10 à 17 % en poids, la teneur en glucides est de 5 à 21 % en poids, la teneur en cendres est de 7 à 13 % en poids, et la teneur en fibres est de 20 à 32 % en poids.

**3.** Composition analogue au son de riz selon la revendication 1 ou la revendication 2, dans laquelle la teneur en cire n'est pas de plus de 1,1 % en poids et la teneur en acides gras libres est de 0,5 à 5 % en poids.

**4.** Aliment pour une utilisation dans la réduction ou la suppression d'une augmentation de la graisse viscérale, l'aliment contenant la composition analogue au son de riz selon l'une quelconque des revendications 1 à 3.

EP 2 172 116 B1

FIG. 1

```
┌─────────────────────────────────────┐
│   Measuring the weight of rice bran  │
└─────────────────────────────────────┘
                    │
┌─────────────────────────────────────┐
│           Drying rice bran           │
└─────────────────────────────────────┘
                    │
┌─────────────────────────────────────┐
│          Roasting rice bran          │
└─────────────────────────────────────┘
                    │
┌─────────────────────────────────────┐
│   Pressing rice bran at low temperature   │
└─────────────────────────────────────┘
        │                       │
┌──────────────────┐   ┌──────────────────────┐
│ Defatted rice bran│   │  Crude oil of rice bran │
└──────────────────┘   └──────────────────────┘
        │                       │
┌──────────────────────┐  ┌──────────────────────────┐
│Roasting defatted rice bran│  │Removing impurities (Filtration│
└──────────────────────┘  │after allowing to stand)     │
        │                 └──────────────────────────┘
┌──────────────────────┐          │
│Cracking defatted rice bran│  ┌──────────────────────┐
└──────────────────────┘  │      Degumming        │
        │                 └──────────────────────┘
┌──────────────────┐              │
│      Cooling      │      ┌──────────────────────┐
└──────────────────┘      │      Dewaxing         │
        │                 └──────────────────────┘
┌──────────────────┐              │
│      Milling      │      ┌──────────────────────┐
└──────────────────┘      │     Decoloring        │
        │                 └──────────────────────┘
┌──────────────────┐              │
│      Sorting      │      ┌──────────────────────┐
└──────────────────┘      │    Deacidifying       │
        │                 └──────────────────────┘
        │                         │
        │                 ┌──────────────────────┐
        │                 │     Wintering         │
        │                 └──────────────────────┘
        │                         │
┌─────────────────────────────────────┐
│                Mixing                │
└─────────────────────────────────────┘
                    │
┌─────────────────────────────────────┐
│      Rice bran-like composition      │
└─────────────────────────────────────┘
```

16

FIG. 2

FIG. 3

FIG. 4

FIG. 5

## REFERENCES CITED IN THE DESCRIPTION

### Patent documents cited in the description

- JP 2007068523 A
- JP 2007104962 A
- JP 2005143364 A
- WO 2007060947 A2
- WO 2007029631 A1

### Non-patent literature cited in the description

- **KAHLON T.S. et al.** Cholesterol lowering effects of rice bran and rice bran oil fractions in hypercholesterolemic hamsters. *CEREAL CHEM.,* 1992, vol. 69 (5), 485-489
- Standard Tables of Food Composition